# EUROPEAN PATENT APPLICATION

(11) **EP 1 913 861 A1**
(43) Date of publication of application: **23.04.2008**
(21) Application number: 06122570.2
(22) Date of filing: 19.10.2006
(51) Int. Cl.: A47L 15/50, A47L 19/04, A61L 2/00

(54) **Dishcloth sanitizing support**

(71) Applicant: Advance Design Ltd., Hong Kong (HK)
(72) Inventor: Eriksen, Steen Mandsfelt, 3230 Græsted (DK)
(74) Representative: Holme Patent A/S

(57) **Abstract**

A dishcloth (1) has a shape like a tube. A support (3) is adapted to elastically stretch the tube-shaped dishcloth while being washed and sanitized in a dishwasher. The support is comfortable to insert in the tube-shaped dishcloth before the washing operation and to pull out again afterwards. The assembly of the dishcloth and the support can easily find place in a dishwasher designed for common kitchen utensils. The support has in itself very small space requirements.

## Description

The invention relates to a dishcloth and a support for the dishcloth, which has a shape like a tube, and also to a method for sanitizing the dishcloth.

It is well known that dishcloths used in e.g. kitchens will be extremely contaminated by bacteria to a degree that could be hazardous to the health of the occupier and visitors to the resident in question.

Dangerous bacteria like *E. coli, Campylobacter* and *Salmonella* can be found in enormous quantities in a dishcloth shortly after using the dishcloth for the first time.

Eating food infected by bacteria from dishcloths infects very many people every year and even if the dishcloths are frequently washed in e.g. a kitchen sink.

US Patent No. 2003202902 provides a solution to this problem by proposing to use a sanitizing frame for keeping a contaminated dishcloth in a stretched condition in which the dishcloth then is washed and dried in a dishwasher.

The high temperature and long retention time which is possible in the dishwasher ensure that the bacteria are effectively killed.

The known sanitizing frame is however relatively large and therefore inconvenient to handle in a normal kitchen where more frames are required if at least one sanitized dishcloth always is to be on hand.

Another drawback is that it is difficult and time consuming to place a dishcloth in the known frame and afterwards removing it again.

Moreover, it is difficult to find place for such frames in normal dishwashers designed for common kitchen utensils.

The above-mentioned disadvantages of the prior art are according to the present invention remedied by,
in a first aspect of the invention providing a dishcloth, a support for the dishcloth and a method for sanitizing the dishcloth of the kind mentioned in the opening paragraph by means of which bacteria in a contaminated dishcloth effectively can be killed,
in a second aspect of the invention providing a dishcloth, a support for the dishcloth and a method for sanitizing the dishcloth of the kind mentioned in the opening paragraph which is easy and comfortable to handle,
in a third aspect of the invention providing a dishcloth, a support for the dishcloth and a method for sanitizing the dishcloth of the kind mentioned in the opening paragraph in which the support has small space requirements,
in a fourth aspect of the invention providing a dishcloth, a support for the dishcloth and a method for sanitizing the dishcloth of the kind mentioned in the opening paragraph in which a dishcloth stretched by the support easily can find place in a dishwasher designed for kitchen utensils,
in a fifth aspect of the invention providing a dishcloth, a support for the dishcloth and a method for sanitizing the dishcloth of the kind mentioned in the opening paragraph in which the support occupy less space than hitherto known.

The novel and unique features of the invention whereby these features are achieved consist in the fact that the support is adapted to elastically stretch the tube-shaped dishcloth.

This implies that a contaminated dishcloth quickly and easily can be stretched by the support and then be placed in a dishwasher for being washed so that bacteria in the dishcloth effectively can be killed.

Dishcloths are made of a flexible material and therefore can be formed as desired. By the term tube-shaped should in the context of the invention be understood that the wall of the dishcloth, seen in section, extends along an endless curve.

The tube-shaped dishcloth according to the invention has an outer and inner surface and therefore the double of the surface of a normal dishcloth covering the same area. Thereby, it is advantageously obtained that the inside of the dishcloth can be turned out and present a less or even not yet contaminated surface so that the dishcloth is serviceable for a longer time than normal before needing to be sanitized.

The tube-shaped dishcloth can be open in both ends or closed in one end and it can be used with a hand inserted or as a normal dishcloth.

In a preferred embodiment according to the invention, the support can be a spring which is formed like a U with two legs and a bottom connecting the two legs.

The spring could e.g. be made of a thread of stainless spring steel.

The spring is formed in such way that a tube-shaped dishcloth can be stretched by the spring force of a spring inserted in the dishcloth which then can be placed in a dishwasher and washed so that bacteria of any kind in the dishcloth can be effectively killed.

The thread of the bottom of the U-formed spring can, according to the invention, be undulated whereby the operator can achieve a firm hold of the spring during inserting and pulling out the spring of a tube-shaped dishcloth.

According to the invention, the spring thread in the legs of the U-formed spring can have an undulating shape with waves extending crosswise of the plane of the U. Thereby, it is obtained that the interior of the tube-shaped dishcloth can be kept open during the washing operation so that the wash water is allowed to flow through the interior of the dishcloth.

The object, features and advantages of the invention will be explained in more details in the following with reference to the drawing in which,
Fig. 1 is a lateral view, seen in cross section, of a dishcloth according to the invention,
Fig. 2 shows the same but taken along the line II-II in fig. 1,
Fig. 3 is a lateral view of a spring according to the invention for stretching the dishcloth shown in figs. 1 and 2,
Fig. 4 shows the same taken along the line IV-IV in fig. 3,
Fig. 5 is a is a lateral view, seen in cross section, of the dishcloth shown in fig. 1 assembled with the spring shown in fig. 3, and
Fig. 6 shows the same taken along the line VI-VI in fig. 5.

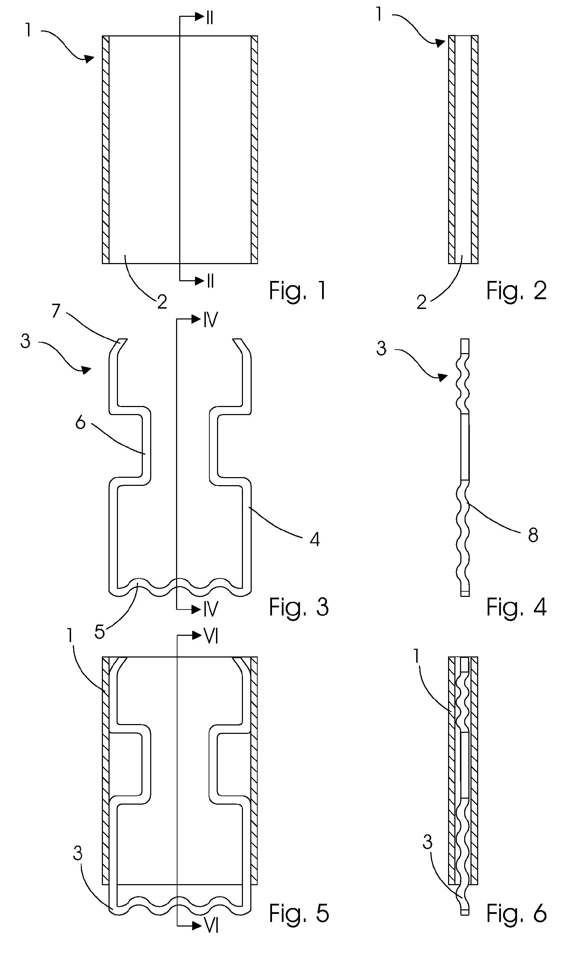

Figs. 1 and 2 show a dishcloth 1 according to the invention. The dishcloth is made of a flexible material having good absorption capacity.

The dishcloth has a shape like a tube, which in fig. 2 is illustrated with a relatively flat shape with a through-going opening 2. In this case, the tube-shaped dishcloth is open in both ends. In another embodiment (not shown), the tube-shaped dishcloth can be closed in one end.

The spring 3 shown in figs. 3 and 4 has in the main a shape like a U with two legs 4 and a bottom 5. In relaxed state, the outer distance between the two legs is somewhat larger than the interior width of the tube-shaped dishcloth in the flattened shape shown in fig. 2.

The two legs each have a part 6 of smaller distance between the two legs than the remainder of the legs. The end parts 7 of the legs are curved inwards. The spring thread in the legs of the U-shaped spring is formed with waves 8 extending crosswise to the plane of the U. The spring thread in the bottom of the U-shaped spring is formed with waves 9 extending into the plane of the U.

In another embodiment (not shown), the U-shaped spring can simply be provided with straight legs and a straight bottom.

In figs. 5 and 6, the spring 3 has been inserted into the dishcloth 1 which now is stretched by the spring force of the spring. Owing to the waves on the legs of the spring the surface of contact between the legs and the tube-shaped dishcloth is so large that it is avoided that the thread of the legs cuts into the material of the dishcloth.

The inwardly curved end parts 7 of the legs facilitate the insertion of the spring. In the beginning of the insertion operation the operator can moreover conveniently squeeze the two legs of the spring together by means of the parts 6 of the legs 4.

The waves 8 of the legs ensure that a through-going passage in the dishcloth is kept open.

The waves 9 of the bottom of the U offer the operator a firm hold of the spring during inserting and pulling out the spring of the tube-shaped dishcloth.

The length of the U-shaped spring is somewhat longer than the length of the tube-shaped dishcloth and in figs. 5 and 6, and the spring is protruding from dishcloth so that a free part is available for inserting the spring into and pulling the spring out of the tube-shaped dishcloth.

The assembly of the dishcloth and spring shown in figs. 5 and 6 is now ready for being placed in a dishwasher (not shown). The assembly of the dishcloth and spring is placed in the same thread rack which is used for holding plates to be washed.

The waves of the bottom of the spring also secure the assembly placed in the thread rack against being moved crosswise out of the rack during the washing operation.

When being inserted into the dishwasher the assembly can run through a normal washing programme where the dishcloth is subjected to such a great heat that bacteria in the dishcloth effectively are killed.

Also the inner surface of the tube-shaped dishcloth is thoroughly sanitized owing to the waves of the legs of the spring keeping a through-going passage open for flow of wash water through the tube-shaped dishcloth.

After the washing process the dishcloth is dried in the dishwasher.

Optionally can the assembly of the dishcloth and spring be further dried outside the dishwasher. A number of washed assemblies could e.g. be placed in a rack or hanged on a rail for further drying.

The sanitized and dried dishcloth can be used immediately after having removed the spring.

The dishcloths can be washed together with other kitchen utilities by using normal detergents.

The dishcloths also can be washed without any detergent but in hot water only.

The spring occupies very little space and many springs can therefore easily be stored in e.g. a drawer in a kitchen.

## Claims

1. A dishcloth and a support (3) for the dishcloth (1), which has a shape like a tube, **characterized in that** the support is adapted to elastically stretch the tube-shaped dishcloth.

2. A dishcloth according to claim 1, **characterized in that** the support is a spring (3) which in the main is formed like a U with two legs (4) and a bottom (5) connecting the two legs.

3. A dishcloth according to claim 2, **characterized in that** the distance between at least a part of the two legs (4) of the U-formed spring (3) in relaxed state is larger than the interior width of the tube-shaped dishcloth (1) in flattened state.

4. A dishcloth according to claim 2 or 3, **characterized in that** the distance between other parts (6) of the two legs (4) of the U-formed spring (3) in relaxed state is less than the largest interior width of the tube-shaped dishcloth (1) in flattened state

5. A dishcloth according to claim 2, 3 or 4, **characterized in that** the spring (3) consists of a thread of spring steel.

6. A dishcloth according to any of the claims 2 - 5, **characterized in that** the material of the spring thread of the U-shaped spring (3) is stainless steel.

7. A dishcloth according to any of the claims 2 - 6, **characterized in that** the spring thread in the bottom (5) of the U-shaped spring (3) has an undulating shape.

8. A dishcloth according to any of the claims 2 - 7, **characterized in that** the spring thread in the legs (4) of the U-shaped spring (3) has an undulating shape with waves (8) extending crosswise of the plane of the U.

9. A dishcloth according to any of tge claims 1 - 8, **characterized in that** the tube-shaped dishcloth (1) is closed in one end.

10. A method for sanitizing the tube-shaped dishcloth (1) of claim 1 - 9 by using the U-shaped spring (3) of claim 2 - 9, **characterized in that** the U-shaped spring (3) is inserted into the tube-shaped dishcloth (1) which then is placed in a dishwasher and washed and dried in this.
